# EUROPEAN PATENT SPECIFICATION

(11) **EP 1 201 233 B1**
(45) Date of publication and mention of the grant of the patent: **08.12.2004**
(21) Application number: 01308949.5
(22) Date of filing: 22.10.2001
(51) Int. Cl.: A61K 9/70, A61K 31/485

(54) **Transdermal dosage form**
Transdermales therapeutisches System
Système thérapeutique transdermique

(30) Priority: 25.10.2000 GB 0026137
(43) Date of publication of application: 02.05.2002
(73) Proprietor: Euro-Celtique S.A., 2330 Luxembourg (LU)
(72) Inventor: Leslie, Stewart Thomas, Cambridge CB2 2RA, Cambridgeshire (GB)
(74) Representative: Ruffles, Graham Keith

(56) References cited:
- WO-A-90/04965
- WO-A-99/16428
- US-A- 5 149 538
- US-A- 5 656 286

## Description

The present invention relates to compositions for the percutaneous administration of opioid analgesics to the human body, and to percutaneous delivery systems such as transdermal patches incorporating such compositions.

Opioid analgesics are widely used in the treatment of moderate to severe pain, especially in terminal cancer or postoperative pain. The most widely used dosage forms of such opioid analgesics is tablet or injection. Tablets are the most favoured form because of ease of administration and convenience for domiciliary treatment. Controlled release tablets enabling once or twice a day dosing of for instance, morphine, oxycodone and hydromorphone are of increasing importance.

A number of proposals have been recently made for the administration of opioid analgesics by percutaneous absorption, using a so called transdermal patch.

Suggested opioid analgesics for this purpose are for instance, morphine, hydromorphone and buprenorphine, see for instance US Patent No. 5069 909, WO 94/10987, JP-A 2-191214.

One disadvantage common to all dosage forms of narcotic opioids is that of drug abuse. Most abusers, in order to obtain the desired effect of an high, usually require a large single dose of drug, normally by bolus injection. As a result of this techniques have been developed by abusers for the extraction of the active principle from the, usually, solid dosage form into aqueous and/or alcoholic solutions. However, because of the various excipients used in solid dosage forms this extraction is neither quick nor simple and serves to some degree as a disincentive.

The development of transdermal patches containing drugs which may be attractive to abusers gives rise to the problem of achieving formulations which will not be convenient to be used as source of the drug for the abuser. In some designs of transdermal patches the drug is provided in solution in a reservoir which might be simply extracted by a hypodermic needle; in other designs the drug may be provided in a gel which may also be susceptible to easy abstraction and then dissolution of the drug into an aqueous and/or alcoholic solution. US Patent No. 5149538 describes a misuse-resistive dosages form containing an opioid and an antagonist for the opioid. WO 90/04965 describes compositions and dosage forms containing an abusable substance and an antogonist of the abusable substance.

An aim of the present invention is to provide a solution to the foregoing problem by providing a composition for the percutaneous administration of an opioid analgesic, preferably a narcotic opioid analgesic, which includes the provision of a component susceptible to co-extraction with the opioid analgesic and which, when administered orally or by injection will distress the abuser. The distressing substance is atropine or an ergolide or a pharmaceutically acceptable salt thereof. Alternatively, the distressing substance is ipecacaunha or a derivative thereof.

According to one aspect of the present invention there is provided a composition for the percutaneous administration of an opioid analgesic, preferably a narcotic opioid analgesic, which comprises a therapeutic amount of the opioid analgesic in association with a vehicle or means for providing a transdermal flux of the opioid analgesic when applied to a human body surface or membrane and quantity of the distressing substance which, when ingested orally or as parenteral bolus injection together with the opioid analgesic will produce a distressful reaction in the recipient.

By distressful reaction is meant a reaction such as vomiting, severe headache or other reaction that will tend to create an aversion to any further attempt to use a similar composition as a source of drug for abuse.

The distressing substance may be any substance which will achieve the distressful reaction when dosed orally or by parenteral bolus injection in an amount which it is convenient to incorporate into a transdermal delivery device.

The distressing substance, may for example, be an ergolide. Among the substances which may be used are for example ergolides such as bromocriptin, lisoline, pergolide, lysuride and their salts such as the maleates, and emetics such as Ipecacuanha. Other suitable substances include atropine or salts thereof.

The distressing substance, if it is non-permeant may be incorporated in the same vehicle as the opioid analgesic, and the vehicle may include a penetration enhancer.

In order to check whether the distressing substance is likely to be non-permeant the formulation can be tested in the donor phase in *in vitro* experiments employing a standard Franz cell arrangement with a suitable circulating recipient phase which may be buffered to e.g. pH 7.4 and a layer of human stratum corneum as barrier. The amount of drug and distressing substance which is absorbed through the barrier into the recipient phase can be observed by determination of the concentration in the recipient phase using HPLC testing of that phase. The test parameters can be readily optimised by the skilled person for the opioid analgesic which is being formulated and the test will then enable the selection of a distressing substance which is non-permeating under those conditions.

The opioid analgesic may be chosen from e.g., morphine, hydromorphone, buprenorphine, ketamine, fentanyl, tramadol or pharmaceutically acceptable, percutaneously transmissible salts thereof.

The compositions may incorporate the opioid analgesics in any vehicle conventionally used in transdermal compositions; thus it may, for example be dissolved in an aqueous and/or alcoholic solution, or incorporated in a suitable matrix including a pressure sensitive adhesive and will usually include a penetration enhancer, or it may be incorporated in a material consisting substantially of penetration enhancer.

The composition will be incorporated in a transdermal device which may be of conventional form.

The device may, accordingly be an adhesive matrix type patch and comprise an impermeable backing layer, a matrix layer which contains the opioid analgesic and a penetration enhancer and distressing substance. The matrix layer may be a microporous material impregnated with the opioid analgesic penetration enhancer and distressing substance. A variety of polymers which can be used as a microporous matrix are known and include a microporous polypropylene such as Celgard sold by Celanese Corp., Charlotte, N.C., USA might be used.

Alternatively, the device may be of the reservoir type. Such devices are taught for example in US Patent Nos. 4379 454 and 4943 435. Devices of this type include an impermeable backing layer, an analgesic reservoir, a drug permeable membrane and an adhesive layer. In devices of this kind the distressing substance may be incorporated in the reservoir together with the opioid analgesic provided it is non-permeant through the drug permeable membrane or is non-permeant through human skin.

Yet a further type of patch is a monolithic system in which a non-porous matrix is swollen with dissolved penetration enhancer, opioid analgesic and distressing agent. The choice of monolithic carrier or vehicle depends on the penetration enhancer and the materials which might be used are, for example acrylate and methacrylate copolymers. Monomers of these materials such as hydroxy ethyl methacrylate dissolved in a mixture of opioid analgesic, penetration enhancer and distressing agent can be polymerised by a suitable free radical polymerisation reaction to yield a cross-linked gel containing drug and enhancer.

Suitable transdermal devices, which may be modified in accordance with the present inventive concept include those described in for example EP-0 577 622, WO 87/06144, WO 94/02119 and US 5375645.

The amount of opioid analgesic included in a transdermal device according to the invention will depend upon the blood levels of the opioid which are desired for adequate analgesic effect, the transdermal flux and the desired duration of action of the device. The determination of these amounts are known from the literature or can readily be found by the skilled person.

The amount of distressing substance, to be incorporated in a transdermal device according to the invention as indicated above, is determined by the requirement that it will be sufficient to cause distress to an abuser to whom it is administered orally or, as appropriate by percutaneous bolus injection. For instance in the case of the ergolides an amount in the range of 5-10mg or more may be suitable, and in the cause of Ipecacuanha 0.1 to 2mg may be suitable. Suitable amounts of these components can be readily determined by suitable trials and experiments.

In preferred embodiments the distressing substance is chosen to be non-permeant either through a membrane which is permeable to the opioid analgesic or non-permeant through human skin. In other preferred embodiments of a transdermal device the distressing substance is separated from the opioid analgesic or is contained in a separate compartment the location of the distressing agent, however being such that in order to remove the opioid analgesic from the device the distressing substance must also be released therewith.

In one preferred embodiment, a composition or device according to the invention contains buprenorphine or pharmaceutically acceptable salt thereof as the opioid analgesic and atropine or pharmaceutically acceptable salt thereof, or an ergolide or pharmaceutically acceptable salt thereof or ipecacuanha as the distressing substance.

## Claims

1. A composition for the percutaneous administration of an opioid analgesic which includes a substance susceptible to co-extraction with the opioid analgesic and which, when administered orally or by injection after extraction by an abuser will distress the abuser, the distressing substance being atropine or an ergolide or a pharmaceutically acceptable salt thereof or ipecacuanha.

2. A composition according to claim 1 which comprises a therapeutic amount of the opioid analgesic in association with a vehicle or means for providing a transdermal flux of the opioid analgesic when applied to a human body surface or membrane and a quantity of the distressing substance which, when ingested orally or as parenteral bolus injection together with the opioid analgesic will produce the distressful reaction in the recipient.

3. A composition according to claim 1 or 2, wherein the distressing substance is an ergolide chosen from bromocriptin, lisoline, pergolide, lysuride or a salt thereof.

4. A composition according to any preceding claim, wherein the distressing substance is non-permeant and is incorporated in a vehicle being the same vehicle as for the opioid analgesic.

5. A composition according to claim 4, wherein the vehicle includes a penetration enhancer.

6. A composition according to any preceding claim, wherein the opioid analgesic is chosen from morphine, hydromorphone, buprenorpbine, ketamine, fentanyl, tramadol, or pharmaceutically acceptable and percutaneously transmissible salts thereof.

7. A composition according to any preceding claim wherein the opioid analgesic is a narcotic opioid analgesic.

8. A composition according to any preceding claim, wherein the opioid analgesic is in an aqueous and/or alcoholic solution, or incorporated in a matrix including a pressure sensitive adhesive.

9. A transdermal device containing a composition according to any preceding claim.

10. A device according to claim 9 which is an adhesive matrix patch and comprises an impermeable backing layer, a matrix layer which contains the opioid analgesic and a penetration enhancer and distressing substance.

11. A device according to claim 9 which is a reservoir device.

12. A device according to claim 9 which is monolithic patch.

13. A composition according to any of claims 1 to 8 or a device according to any of claims 9 to 12 which contains buprenorphine or pharmaceutically acceptable salt thereof as the opioid analgesic and atropine or pharmaceutically acceptable salt thereof, or an ergolide or pharmaceutically acceptable salt thereof or ipecacuanha as the distressing substance.

## Patentansprüche

1. Zusammensetzung für eine perkutane Verabreichung eines Opioid-Analgetikums, welche eine Substanz enthält, welche geeignet ist für eine gemeinsame Extraktion mit dem Opioid-Analgetikum und welche, wenn sie nach der Extraktion von einer für eine missbräuchliche Anwendung verantwortlichen Person oral oder durch eine Injektion verabreicht wird, bei der für die missbräuchliche Anwendung verantwortlichen Person einen Schmerzen hervorrufen wird, wobei die einen Schmerzen hervorrufende Substanz aus Atropin oder aus einem Ergolid oder aus einem pharmazeutisch annehmbaren Salz derselben oder aus einem Ipecacuanha besteht.

2. Zusammensetzung gemäß Anspruch 1, welche als Inhalt aufweist; eine therapeutische Menge des Opioid-Analgetikums in Verbindung mit einem Transportmittel oder mit einem Hilfsmittel für die Bereitstellung eines transdermalen Flusses des Opioid-Analgetikums, wenn dieselbe auf die Oberfläche oder auf eine Membran des menschlichen Körpers aufgetragen wird, und eine gewisse Menge der einen Schmerzen hervorrufenden Substanz, welche, wenn sie oral oder als eine parenterale Bolusinjektion zusammen mit dem Opioid-Analgetikum aufgenommen wird, die den Schmerzen hervorrufende Reaktion bei dem Empfänger auslösen wird.

3. Zusammensetzung gemäß Anspruch 1 oder 2, in welcher die den Schmerzen hervorrufende Substanz aus einem Ergolid besteht, welches ausgewählt wird unter Bromocriptin, Lisolin, Pergolid, Lysurid oder einem Salz derselben.

4. Zusammensetzung gemäß irgendeinem der vorhergehenden Ansprüche, in welcher die den Schmerzen hervorrufende Substanz nicht durchdringend ist und in einem Transportmittel eingebunden ist, welches dasselbe Transportmittel ist wie für das Opioid-Analgetikum.

5. Zusammensetzung gemäß Anspruch 4, in welcher das Transportmittel ein Verstärkungsmittel für die Penetration enthält.

6. Zusammensetzung gemäß irgendeinem der vorhergehenden Ansprüche, in welcher das Opioid-Analgetikum ausgewählt wird unter Morphin, Hydromorphon, Buprenorphin, Ketamin, Fentanyl, Tramadol oder pharmazeutisch annehmbaren und perkutan übertragbaren Salzen derselben.

7. Zusammensetzung gemäß irgendeinem der vorhergehenden Ansprüche, in welcher das Opioid-Analgetikum aus einem narkotischen Opioid-Analgetikum besteht.

8. Zusammensetzung gemäß irgendeinem der vorhergehenden Ansprüche, in welcher das Opioid-Analgetikum sich in einer wässrigen und/oder alkoholischen Lösung befindet, oder in einer Matrix eingebunden ist, welche ein druckempfindliches Klebemittel enthält.

9. Transdermale Einrichtung, welche eine Zusammensetzung gemäß irgendeinem der vorhergehenden Ansprüche enthält.

10. Einrichtung gemäß Anspruch 9, welche aus einem haftenden Matrixheftpflaster besteht und welche enthält; eine undurchlässige Verstärkungsschicht, eine Matrixschicht, welche das Opioid-Analgetikum aufweist, und ein Verstärkungsmittel für die Penetration sowie eine die Schmerzen hervorrufende Substanz.

11. Einrichtung gemäß Anspruch 9, welche aus einer Reservoirvorrichtung besteht.

12. Einrichtung gemäß Anspruch 9, welche aus einem monolithisches Pflaster besteht.

13. Zusammensetzung gemäß irgendeinem der Ansprüche von 1 bis 8 oder Einrichtung gemäß irgendeinem der Ansprüche von 9 bis 12, welche enthält; Buprenorphin oder ein pharmazeutisch annehmbares Salz desselben als das Opioid-Analgetikum und Atropin oder ein pharmazeutisch annehmbares Salz desselben, oder ein Ergolid oder ein pharmazeutisch annehmbares Salz desselben oder Ipecacuanha als die einen Schmerzen hervorrufende Substanz.

## Revendications

1. Composition pour l'administration percutanée d'un analgésique opioïde qui inclut une substance de nature à être co-extraite avec l'analgésique opioïde et qui, lorsqu'elle est administrée oralement ou par injection après extraction par un toxicomane, entraînera des troubles sur le toxicomane, la substance entraînant des troubles étant l'atropine ou un ergolide ou un sel pharmaceutiquement acceptable de ceux-ci ou l'ipécacuanha.

2. Composition suivant la revendication 1, qui comprend une quantité thérapeutique de l'analgésique opioïde en association avec un véhicule ou un moyen pour donner un flux transdermique de l'analgésique opioïde lorsqu'il est appliqué à une surface ou une membrane du corps humain et une quantité de la substance entraînant des troubles qui, lorsqu'elle est ingérée oralement ou comme une injection parentérale d'un bolus accompagnée de l'analgésique opioïde, produira la réaction entraînant des troubles chez le receveur.

3. Composition suivant la revendication 1 ou 2, dans laquelle la substance entraînant des troubles est un ergolide choisi parmi la bromocriptine, la lisoline, le pergolide, le lysuride ou un sel de ceux-ci.

4. Composition suivant l'une quelconque des revendications précédentes, dans laquelle la substance entraînant des troubles est non pénétrante et elle est incorporée dans un véhicule qui est le même véhicule que pour l'analgésique opioïde.

5. Composition suivant la revendication 4, dans laquelle le véhicule inclut une substance augmentant la pénétration.

6. Composition suivant l'une quelconque des revendications précédentes, dans laquelle l'analgésique opioïde est choisi parmi la morphine, l'hydromorphone, la buprénorphine, la kétamine, le fentanyl, le tramadol ou des sels pharmaceutiquement acceptables et transmissibles par voie percutanée de ceux-ci.

7. Composition suivant l'une quelconque des revendications précédentes, dans laquelle l'analgésique opioïde est un analgésique opioïde narcotique.

8. Composition suivant l'une quelconque des revendications précédentes, dans laquelle l'analgésique opioïde est dans une solution aqueuse et/ou alcoolique ou incorporé dans une matrice incluant un adhésif sensible à une pression.

9. Dispositif transdermique contenant une composition suivant l'une quelconque des revendications précédentes.

10. Dispositif suivant la revendication 9, qui est un patch à matrice adhésive et comprend une couche support imperméable, une couche de matrice qui contient l'analgésique opioïde et une substance augmentant la pénétration et la substance entraînant des troubles.

11. Dispositif suivant la revendication 9, qui est un dispositif à réservoir.

12. Dispositif suivant la revendication 9, qui est un patch monolithique.

13. Composition suivant l'une quelconque des revendications 1 à 8 ou dispositif suivant l'une quelconque des revendications 9 à 12, qui contient de la buprénorphine ou un sel pharmaceutiquement acceptable de celle-ci en tant qu'analgésique opioïde et de l'atropine ou un sel pharmaceutiquement acceptable de celle-ci ou un ergolide ou un sel pharmaceutiquement acceptable de celui-ci ou de l'ipécacuanha en tant que substance entraînant des troubles.
